# EUROPÄISCHE PATENTANMELDUNG

(11) **EP 4 789 696 A1**
(43) Veröffentlichungstag der Anmeldung: **12.08.2026**
(21) Anmeldenummer: 26156254.0
(22) Anmeldetag: 04.02.2026
(51) Int. Cl.: A61M 1/14, G01D 11/30

(54) **HALTEVORRICHTUNG ZUR HALTERUNG EINES SENSORELEMENTS AN EINEM DIALYSEGERÄT**

(30) Priorität: 11.02.2025 DE 102025104880
(71) Anmelder: B. Braun Avitum AG, 34212 Melsungen (DE)
(72) Erfinder: Horschke, Sebastian, 37235 Hessisch Lichtenau (DE)
(74) Vertreter: Patentanwälte Ruff, Wilhelm, Beier, Dauster & Partner mbB

(57) **Zusammenfassung**

Die Erfindung betrifft eine Haltevorrichtung zur Halterung eines Sensorelements an einem Dialysegerät, die Haltevorrichtung aufweisend einen Befestigungsabschnitt, der zum Befestigen der Haltevorrichtung an dem Dialysegerät eingerichtet ist, einen Hohlzylinderabschnitt, der entlang einer Längsachse zwischen einem ersten Hohlzylinderende und einem zweiten Hohlzylinderende längserstreckt ist, und eine axiale Haltebohrung sowie einen Einführschlitz aufweist, wobei das erste Hohlzylinderende mit dem Befestigungsabschnitt verbunden ist, wobei die Haltebohrung zum Aufnehmen des Sensorelements eingerichtet ist, wobei der Einführschlitz unter Ausbildung eines in Umfangsrichtung offenen C-förmigen Querschnitts des Hohlzylinderabschnitts zwischen dem ersten Hohlzylinderende und dem zweiten Hohlzylinderende durchgängig längserstreckt ist und radial in die Haltebohrung mündet, und wobei der Hohlzylinderabschnitt wenigstens abschnittsweise elastisch nachgiebig ist, wodurch das Sensorelement durch den Einführschlitz radial in die Haltebohrung einbringbar ist.

## Beschreibung

Die Erfindung betrifft eine Haltevorrichtung zur Halterung eines Sensorelements an einem Dialysegerät.

Dialysegeräte sind üblicherweise mit Sensorelementen ausgestattet, im Speziellen mit Reedsensoren die auch als Reedschalter bezeichnet werden.

Aufgabe der Erfindung ist es, eine vereinfachte Montage und Demontage eines Sensorelements an einem Dialysegerät zu ermöglichen.

Diese Aufgabe wird durch das Bereitstellen einer Haltevorrichtung mit den Merkmalen des Anspruchs 1 gelöst. Vorteilhafte Weiterbildungen sind in den Unteransprüchen angegeben. Der Wortlaut der Ansprüche wird durch Bezugnahme zum Gegenstand der Beschreibung gemacht.

Die erfindungsgemäße Haltevorrichtung ist zur Halterung eines Sensorelements, vorzugsweise eines Reedsensors, an einem Dialysegerät vorgesehen und weist einen Befestigungsabschnitt und einen Hohlzylinderabschnitt auf. Der Befestigungsabschnitt ist zum Befestigen der Haltevorrichtung an dem Dialysegerät eingerichtet. Der Hohlzylinderabschnitt ist entlang einer Längsachse zwischen einem ersten Hohlzylinderende und einem zweiten Hohlzylinderende längserstreckt. Der Hohlzylinderabschnitt weist eine axiale Haltebohrung und einen Einführschlitz auf. Das erste Hohlzylinderende ist mit dem Befestigungsabschnitt verbunden. Die Haltebohrung ist zum Aufnehmen des Sensorelements eingerichtet. Der Einführschlitz ist unter Ausbildung eines in Umfangsrichtung offenen C-förmigen Querschnitts des Hohlzylinderabschnitts zwischen dem ersten Hohlzylinderende und dem zweiten Hohlzylinderende durchgängig längserstreckt. Der Einführschlitz mündet radial in die Haltebohrung. Der Hohlzylinderabschnitt ist wenigstens abschnittsweise elastisch nachgiebig. Dadurch ist das Sensorelement durch den Einführschlitz radial in die Haltebohrung einbringbar. Die erfindungsgemäße Haltevorrichtung ermöglicht eine vereinfachte Montage und Demontage des Sensorelements. Zwecks Montage wird zunächst die Haltevorrichtung mittels des Befestigungsabschnitts an dem Dialysegerät befestigt. Das Sensorelement wird radial durch den Einführschlitz in die Haltebohrung eingeführt. Da der Hohlzylinderabschnitt wenigstens abschnittsweise elastisch nachgiebig ist, gibt der Einführschlitz beim Einführen des Sensorelements unter Einwirkung desselben elastisch nach, weitet sich in Umfangsrichtung und federt nach dem Einführen des Sensorelements elastisch zurück. In eingeführten Zustand ist das Sensorelement kraft- und/oder formschlüssig in der Haltebohrung gehalten. In eingeführtem Zustand bildet der Hohlzylinderabschnitt eine Rast-, Klemm- und/oder Clipsverbindung mit dem Sensorelement aus. Die Demontage kann in kinematisch umgekehrter Weise erfolgen.

In Ausgestaltung der Erfindung ist die Haltebohrung axial beidseits offen und weist im Bereich des zweiten Hohlzylinderendes wenigstens einen Vorsprung auf, der radial nach innen ragt. Durch den wenigstens einen radial nach innen ragenden Vorsprung wird ein axiales Herausziehen des Sensorelements aus der Haltebohrung verhindert oder wenigstens erschwert. Durch die beidseits offene Gestaltung der Haltebohrung kann eine etwaige Signalleitung des Sensorelements auf besonders einfache Weise einends aus der Haltebohrung herausgeführt werden. Die auch andernends offene Gestaltung der Haltebohrung kann eine verbesserte Funktion des Sensorelements ermöglichen.

In weiterer Ausgestaltung der Erfindung weist der Hohlzylinderabschnitt im Bereich des zweiten Hohlzylinderendes seitlich in den Einführschlitz ragende und elastisch nachgiebige Klemmabschnitte auf, die zum elastischen Klemmen des Sensorelements eingerichtet sind. Die Klemmabschnitte bilden gleichsam Endabschnitte des C-förmigen Querschnitts und liegen einander in Umfangsrichtung gegenüber. Im Bereich der Klemmabschnitte ist der Einführschlitz schmaler als abseits der Klemmabschnitte. Die Klemmabschnitte bilden eine abschnittsweise Berandung des Einführschlitzes. Die Klemmabschnitte können auch als Klemmkanten des Einführschlitzes bezeichnet werden. Bei einer Ausgestaltung sind die Klemmabschnitte jeweils über 20 % bis 80 %, vorzugsweise über 30 % bis 60 %, weiter vorzugsweise über 40 bis 50 %, einer Gesamtlänge des Hohlzylinderabschnitts längserstreckt.

In weiterer Ausgestaltung der Erfindung weist der Einführschlitz im Bereich des ersten Hohlzylinderendes und damit im Bereich des Befestigungsabschnitts eine Verbreiterung auf. Durch die Verbreiterung, die auch als Freistellung bezeichnet werden kann, wird vermieden, dass das Sensorelement beim Einbringen in die Haltebohrung im Bereich des ersten Hohlzylinderendes und damit auch im Bereich des Befestigungsabschnitts in Kontakt mit dem Einführschlitz, genauer: mit den Einführschlitz berandenden Kanten des Hohlzylinderabschnitts, gelangt. Im Bereich der Verbreiterung werden somit mechanische Spannungsspitzen und damit eine mechanische Überbeanspruchung des Hohlzylinderabschnitts vermieden.

In weiterer Ausgestaltung der Erfindung sind der Hohlzylinderabschnitt und der Befestigungsabschnitt einstückig miteinander verbunden. Durch die einstückige Verbindung kann auf eine gesonderte Fügeverbindung und entsprechende Fügemittel zur Verbindung des Hohlzylinderabschnitts mit dem Befestigungsabschnitt verzichtet werden. Hierdurch wird ein besonders einfacher Aufbau der Haltevorrichtung erreicht. Vorzugsweise ist die Haltevorrichtung einstückig.

In weiterer Ausgestaltung der Erfindung ist die Haltevorrichtung einstückig aus Kunststoff gefertigt. Bei einer Ausgestaltung ist die Haltevorrichtung ein aus Kunststoff gefertigtes Spritzgussbauteil. Bei einer weiteren Ausgestaltung ist eine additive Fertigung vorgesehen, beispielsweise mittels 3D-Druck. Bei einer Ausgestaltung ist der verwendete Kunststoff Polyoxymethylen (POM). Bei einer weiteren Ausgestaltung ist der verwendete Kunststoff Polyamid (PA).

In weiterer Ausgestaltung der Erfindung ist der Hohlzylinderabschnitt mittels einer Verrippung an den Befestigungsabschnitt angeschlossen. Die Verrippung bewirkt eine mechanische Versteifung und wirkt ungewollten elastischen Deformationen entgegen. Die Verrippung kann grundsätzlich auf jede für den vorliegenden Zweck geeignete Weise gestaltet sein.

In weiterer Ausgestaltung der Erfindung weist die Verrippung mehrere Rippen auf, die jeweils ausgehend von dem Befestigungsabschnitt bis zu einer Außenfläche des Hohlzylinderabschnitts axial längserstreckt sind. Mit anderen Worten: Die Rippen sind jeweils einends mit dem Befestigungsabschnitt und andernends mit dem Hohlzylinderabschnitt verbunden. Vorzugsweise sind die Rippen jeweils entlang der Längsachse des Hohlzylinderabschnitts längserstreckt, im Speziellen parallel zur Längsachse.

In weiterer Ausgestaltung der Erfindung weist die Verrippung mehrere Rippen auf, die in Umfangsrichtung des Hohlzylinderabschnitts zueinander versetzt angeordnet sind. Vorzugsweise sind die mehreren Rippen - in Übereinstimmung mit der vorhergehenden Ausgestaltung - jeweils ausgehend von dem Befestigungsabschnitt bis auf eine Außenfläche des Hohlzylinderabschnitts axial längserstreckt. Durch die in Umfangsrichtung versetzte Anordnung der mehreren Rippen ergeben sich vorteilhafte mechanische Eigenschaften.

In weiterer Ausgestaltung der Erfindung ist der Hohlzylinderabschnitt mittels einer Verrundung an den Befestigungsabschnitt angeschlossen. Die Verrundung ist im Bereich des ersten Hohlzylinderendes angeordnet. Die Verrundung wirkt einer Kerbwirkung und damit mechanischen Spannungsspitzen entgegen. Durch die Verrundung weist der Hohlzylinderabschnitt im Bereich des ersten Hohlzylinderendes einen größeren Außendurchmesser auf als im Bereich des zweiten Hohlzylinderendes.

In weiterer Ausgestaltung der Erfindung ist die Verrippung auf der Verrundung angeordnet. Durch das Anordnen der Verrippung auf der Verrundung wird eine in mechanischer Hinsicht besonders vorteilhafte Gestaltung der Haltevorrichtung erreicht.

In weiterer Ausgestaltung der Erfindung weist der Befestigungsabschnitt wenigstens einen Flanschabschnitt auf, der radial von dem zweiten Hohlzylinderende abragt und wenigstens eine Befestigungsbohrung zum Aufnehmen eines Befestigungsmittels aufweist. Vorzugsweise ist der wenigstens eine Flanschabschnitt orthogonal zur Längsachse des Hohlzylinderabschnitts angeordnet. Vorzugsweise ist die Befestigungsbohrung zum Aufnehmen einer Befestigungsschraube eingerichtet. Bei einer bevorzugten Ausgestaltung weist der Befestigungsabschnitt zwei Flanschabschnitte auf, die an radial gegenüberliegenden Seiten angeordnet sind. Mit anderen Worten: Die beiden Flanschabschnitte sind um 180° um die Längsachse des Hohlzylinderabschnitts zueinander versetzt angeordnet.

Die Erfindung betrifft zudem eine Anordnung mit einer Haltevorrichtung gemäß der vorhergehenden Beschreibung und mit einem Sensorelement, das in der Haltebohrung der Haltevorrichtung gehalten ist.

Die Erfindung betrifft zudem ein Dialysegerät mit einer Haltevorrichtung gemäß der vorhergehenden Beschreibung. Alternativ oder zusätzlich weist das erfindungsgemäße Dialysegerät eine Anordnung gemäß der vorhergehenden Ausgestaltung auf.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.

Es zeigen:
- Fig. 1: in schematischer Perspektivansicht eine Ausführungsform eines erfindungsgemäßen Dialysegeräts mit einer Ausführungsform einer erfindungsgemäßen Haltevorrichtung,
- Fig. 2: in schematischer Perspektivansicht die Haltevorrichtung des Dialysegeräts nach Fig. 1 und
- Fig. 3: in einem schematischen Längsschnitt die Haltevorrichtung nach Fig. 2 zusammen mit einem mittels der Haltevorrichtung gehaltenen Sensorelement in Form eines Reedsensors.

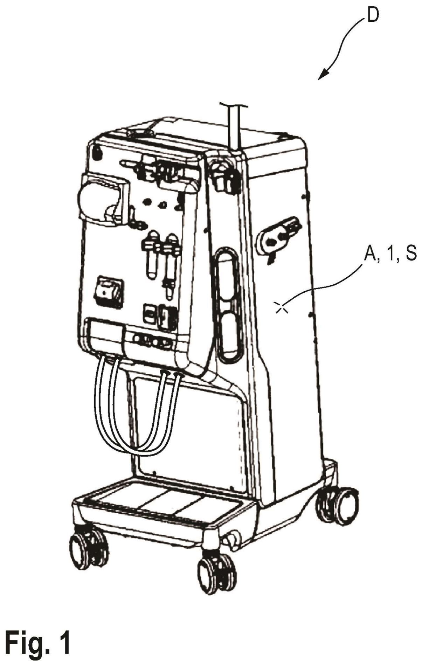

Gemäß Fig. 1 ist ein Dialysegerät D zur extrakorporalen Blutbehandlung vorgesehen. Der grundsätzliche Aufbau und die Funktion des Dialysegeräts D sind der zuständigen Fachperson bekannt. Auf weitere diesbezügliche Einzelheiten muss daher nicht näher eingegangen werden.

Das Dialysegerät D ist mit einem Sensorelement S ausgestattet, das mittels einer Haltevorrichtung 1 gehalten und geräteseitig befestigt ist.

Die Haltevorrichtung 1 und das Sensorelement S bilden gemeinsam eine Anordnung A.

Die Anordnung A ist in Fig. 1 nicht im Detail gezeigt und an einer hierfür vorgesehenen Stelle im Inneren des Dialysegeräts D angeordnet. Dabei spielt die exakte Positionierung der Anordnung A in Bezug auf das Dialysegerät D für die vorliegende Erfindung keine wesentliche Rolle. Insoweit ist die in Fig. 1 gezeigte exemplarische Positionierung der Anordnung A hinter einer Seitenwand eines Gehäuses des Dialysegeräts D als rein exemplarisch zu verstehen.

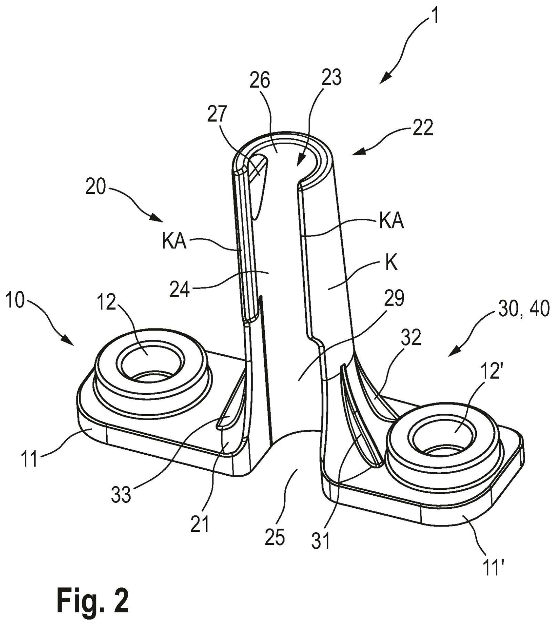

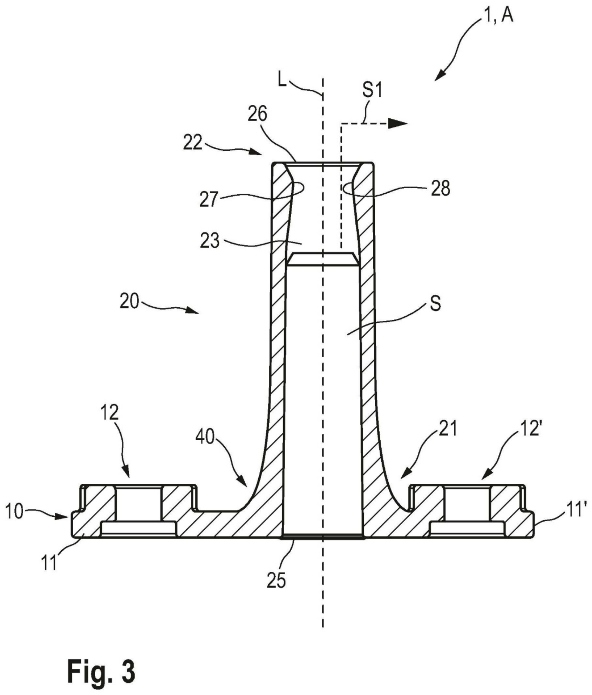

Die Haltevorrichtung 1 ist im Detail in den Fig. 2 und 3 gezeigt und weist einen Befestigungsabschnitt 10 und einen Hohlzylinderabschnitt 20 auf.

Der Befestigungsabschnitt 10 ist zum Befestigen der Haltevorrichtung 1 an dem Dialysegerät D eingerichtet. Der Befestigungsabschnitt 10 weist einen nachfolgend noch näher erläuterten spezifischen Aufbau auf.

Der Hohlzylinderabschnitt 20 ist entlang einer Längsachse L (siehe Fig. 3) zwischen einem ersten Hohlzylinderende 21 und einem zweiten Hohlzylinderende 22 längserstreckt. Der Hohlzylinderabschnitt 20 weist eine Haltebohrung 23 und einen Einführschlitz 24 auf.

Bei der gezeigten Ausführungsform ist das erste Hohlzylinderende 21 mit dem Befestigungsabschnitt 10 verbunden. Diese Verbindung ist auf eine nachfolgend noch näher erläuterte Weise ausgeführt.

Die Haltebohrung 23 ist zum Aufnehmen des besagten Sensorelements S eingerichtet. Der aufgenommene Zustand ist in Fig. 3 schematisch gezeigt.

Bei der gezeigten Ausführungsform ist das Sensorelement S ein Reedsensor. Bei der gezeigten Ausführungsform weist das Sensorelement S eine zylindrische Gestaltung auf. Dabei ist eine Außenkontur des Sensorelements S komplementär zu einer Innenkontur des Hohlzylinderabschnitts 20 und/oder der Haltebohrung 23 gestaltet. In aufgenommenem Zustand ist das Sensorelement S koaxial zu der Längsachse L in der Haltebohrung 23 aufgenommen. Ein Außendurchmesser des Sensorelements S entspricht in etwa einem Innendurchmesser der Haltebohrung 23. Im Speziellen ist der Außendurchmesser des Sensorelements S geringfügig größer als der Innendurchmesser der Haltebohrung 23.

Der Einführschlitz 24 ist unter Ausbildung eines in Umfangsrichtung offenen C-förmigen Querschnitts des Hohlzylinderabschnitts 20 zwischen dem ersten Hohlzylinderende 21 und dem zweiten Hohlzylinderende 22 durchgängig längserstreckt. Der Einführschlitz 24 mündet radial und damit orthogonal zur Längsachse L in die Haltebohrung 23.

Der Hohlzylinderabschnitt 20 ist wenigstens abschnittsweise elastisch nachgiebig. Hierdurch kann das Sensorelement S durch den Einführschlitz 24 radial in die Haltebohrung 23 eingebracht werden. Mit anderen Worten: Das Sensorelement S kann in die Haltebohrung 23 und damit in den Hohlzylinderabschnitt 20 eingerastet oder auch eingeclipst werden. Hierdurch wird eine maßgebliche Vereinfachung der Montage (und Demontage) des Sensorelements S an dem Dialysegerät D erreicht.

Bei der gezeigten Ausführungsform ist der Hohlzylinderabschnitt 20 axial beidseits offen. Demnach weist die Haltebohrung 23 im Bereich des ersten Hohlzylinderendes 21 eine erste Stirnöffnung 25 auf. Im Bereich des zweiten Hohlzylinderendes 22 weist die Haltebohrung 23 eine zweite Stirnöffnung 26 auf. Die beiden Stirnöffnungen 25, 26 liegen einander entlang der Längsachse L gegenüber.

Bei der in Fig. 3 gezeigten exemplarischen Konfiguration ist ein unteres Stirnende des Sensorelements S in etwa bündig mit der ersten Stirnöffnung 25 angeordnet. Ein gegenüberliegendes Stirnende des Sensorelements S ist ausgehend von der zweiten Stirnöffnung 26 axial zurückversetzt.

Um ein unbeabsichtigtes axiales Herausziehen des Sensorelements S über die zweite Stirnöffnung 26 zu verhindern, weist die Haltebohrung 23 im Bereich des zweiten Hohlzylinderendes 22 wenigstens einen Vorsprung 27, 28 auf. Der wenigstens eine Vorsprung 27, 28 ragt radial nach innen. Hierdurch wird ein Innendurchmesser der Haltebohrung 23 im Bereich des zweiten Hohlzylinderendes 22 verjüngt.

Vorliegend ist im Bereich des wenigstens einen Vorsprungs 27, 28 ein Innendurchmesser der Haltebohrung 23 derart kleiner als der Außendurchmesser des Sensorelements, dass auch eine elastische Verformung des Hohlzylinderabschnitts 20 kein axiales Herausziehen des Sensorelements S aus der zweiten Stirnendöffnung 26 erlaubt.

Bei der gezeigten Ausführungsform sind zwei Vorsprünge vorhanden, die auch als erster Vorsprung 27 und zweiter Vorsprung 28 bezeichnet werden können. Die beiden Vorsprünge 27, 28 sind um die Längsachse L und damit in Umfangsrichtung zueinander versetzt angeordnet. Der Versatz beträgt vorliegend 180°.

Bei der gezeigten Ausführungsform weist das Sensorelement S eine Signalleitung S1 auf. Die Signalleitung S1 ist einends, vorzugsweise unlösbar, mit dem Sensorelement S verbunden. Andernends ist die Signalleitung S1 an eine hierfür vorgesehene Einrichtung des Dialysegeräts D anschließbar. Bei der gezeigten Ausführungsform ist die Signalleitung S1 durch die zweite Stirnöffnung 26 aus dem Hohlzylinderabschnitt 20 herausgeführt.

Bei der gezeigten Ausführungsform weist der Hohlzylinderabschnitt 20 im Bereich des zweiten Hohlzylinderendes 22 seitlich in den Einführschlitz 24 ragende und elastisch nachgiebige Klemmabschnitte KA auf, die zum elastischen Klemmen des Sensorelements S eingerichtet sind. Im Bereich der Klemmabschnitte KA ist der Einführschlitz 24 schmaler als abseits der Klemmabschnitte KA. Die Klemmabschnitte KA bilden eine abschnittsweise Berandung des Einführschlitzes 24. Die Klemmabschnitte KA können auch als Klemmkanten bezeichnet werden. Bei der gezeigten Ausführungsform sind die Klemmabschnitte KA jeweils über 50 % einer Gesamtlänge des Hohlzylinderabschnitts 20 längserstreckt.

Bei der gezeigten Ausführungsform weist der Einführschlitz 24 im Bereich des ersten Hohlzylinderendes 21 und damit im Bereich des Befestigungsabschnitts 10 eine Verbreiterung 29 auf. Die Verbreiterung 29 kann auch als Freistellung bezeichnet werden. Bei der gezeigten Ausführungsform erstreckt die Verbreiterung 29 sich in etwa über eine Hälfte einer Gesamtlänge des Hohlzylinderabschnitts 20. Durch die Verbreiterung 29 werden Spannungsspitzen beim radialen Einrasten des Sensorelements S in die Haltebohrung 23 vermieden.

Bei der gezeigten Ausführungsform sind der Hohlzylinderabschnitt 20 und der Befestigungsabschnitt 10 einstückig miteinander verbunden. Im Speziellen ist die gesamte Haltevorrichtung 1 einstückig ausgebildet.

Vorliegend ist die Haltevorrichtung 1 einstückig aus Kunststoff K gefertigt. Als Kunststoff K kommen insbesondere Polyoxymethylen (POM) und/oder Polyamid (PA) infrage. Bei der gezeigten Ausführungsform ist die Haltevorrichtung 1 per Spritzguss gefertigt. Alternativ ist bei einer in den Figuren nicht gezeigten Ausführungsform eine additive Fertigung vorgesehen, beispielsweise mittels 3D-Druck.

Bei der gezeigten Ausführungsform ist der Hohlzylinderabschnitt 20 mittels einer Verrippung 30 an den Befestigungsabschnitt 10 angeschlossen. Die Verrippung 30 bewirkt eine mechanische Versteifung. Die Verrippung 30 ist im Bereich des ersten Hohlzylinderendes 21 ausgebildet.

Bei der gezeigten Ausführungsform weist die Verrippung 30 mehrere Rippen 31, 32, 33 auf, wobei in Fig. 2 aufgrund der dort gezeigten Perspektive lediglich drei Rippen der mehreren Rippen ersichtlich sind. Die mehreren Rippen 31, 32, 33 sind jeweils ausgehend von dem Befestigungsabschnitt 10 bis auf eine Außenfläche des Hohlzylinderabschnitts 20 axial längserstreckt. Bei der gezeigten Ausführungsform ist die Längserstreckung der Rippen 31, 32, 33 parallel zur Längsachse L des Hohlzylinderabschnitts 20.

Die mehreren Rippen 31, 32, 33 sind jeweils einends mit dem Befestigungsabschnitt 10 und andernends mit dem Hohlzylinderabschnitt 20 verbunden.

Bei der gezeigten Ausführungsform sind die mehreren Rippen 31, 32, 33 in Umfangsrichtung des Hohlzylinderabschnitts 20 und damit um die Längsachse L zueinander versetzt angeordnet. Der Versatz ist vorliegend gleichmäßig.

Vorliegend ist der Hohlzylinderabschnitt 20 mittels einer Verrundung 40 an den Befestigungsabschnitt 10 angeschlossen. Die Verrundung 40 ist im Bereich des ersten Hohlzylinderendes 21 umlaufend erstreckt und vermindert eine Kerbwirkung zwischen dem Hohlzylinderabschnitt 20 und dem Befestigungsabschnitt 10. Durch die Verrundung 40 weist der Hohlzylinderabschnitt 20 einends eine nach Art eines Baumstamms im Bereich der Wurzeln aufgeweitete Gestaltung auf.

Bei der gezeigten Ausführungsform ist die Verrippung 30 auf der Verrundung 40 angeordnet. Die sich ergebende Gestaltgebung ist besonders vorteilhaft, insbesondere beanspruchungsgerecht.

Bei der gezeigten Ausführungsform weist der Befestigungsabschnitt 10 wenigstens einen Flanschabschnitt 11 auf. Im Speziellen sind vorliegend zwei Flanschabschnitte 11, 11' vorhanden, die auch als erster Flanschabschnitt 11 und zweiter Flanschabschnitt 11' bezeichnet werden können. Die beiden Flanschabschnitte 11, 11' ragen jeweils radial von dem ersten Hohlzylinderende 21 ab, im Speziellen orthogonal zu der Längsachse L.

Dabei sind die beiden Flanschabschnitte 11, 11' vorliegend um die Längsachse L zueinander versetzt angeordnet. Der Versatz beträgt vorliegend 180°.

Die beiden Flanschabschnitte 11, 11' weisen jeweils eine Befestigungsbohrung 12, 12' auf, die zur Aufnahme eines Befestigungsmittels eingerichtet sind. Als Befestigungsmittel sind vorliegend Befestigungsschrauben vorgesehen.

Die beiden Flanschabschnitte 11, 11' liegen im befestigten Zustand der Haltevorrichtung 1 plan auf einem hierfür vorgesehenen Abschnitt des Dialysegeräts D auf und sind mittels der besagten Befestigungsschrauben mit dem besagten Abschnitt verschraubt.

## Patentansprüche

1. Haltevorrichtung (1) zur Halterung eines Sensorelements (S) an einem Dialysegerät (D), die Haltevorrichtung (1) aufweisend
einen Befestigungsabschnitt (10), der zum Befestigen der Haltevorrichtung (1) an dem Dialysegerät (D) eingerichtet ist,
einen Hohlzylinderabschnitt (20), der entlang einer Längsachse (L) zwischen einem ersten Hohlzylinderende (21) und einem zweiten Hohlzylinderende (22) längserstreckt ist, und eine axiale Haltebohrung (23) sowie einen Einführschlitz (24) aufweist,
wobei das erste Hohlzylinderende (21) mit dem Befestigungsabschnitt (10) verbunden ist,
wobei die Haltebohrung (23) zum Aufnehmen des Sensorelements (S) eingerichtet ist,
wobei der Einführschlitz (24) unter Ausbildung eines in Umfangsrichtung offenen C-förmigen Querschnitts des Hohlzylinderabschnitts (20) zwischen dem ersten Hohlzylinderende (21) und dem zweiten Hohlzylinderende (22) durchgängig längserstreckt ist und radial in die Haltebohrung (23) mündet,
und wobei der Hohlzylinderabschnitt (20) wenigstens abschnittsweise elastisch nachgiebig ist, wodurch das Sensorelement (S) durch den Einführschlitz (24) radial in die Haltebohrung (23) einbringbar ist.

2. Haltevorrichtung (1) nach Anspruch 1, wobei die Haltebohrung (23) axial beidseits offen ist und im Bereich des zweiten Hohlzylinderendes (22) wenigstens einen Vorsprung (27, 28) aufweist, der radial nach innen ragt, um einem axialen Herausziehen des Sensorelements (S) aus der Haltebohrung (23) entgegenzuwirken.

3. Haltevorrichtung (1) nach Anspruch 1 oder 2, wobei der Hohlzylinderabschnitt (20) im Bereich des zweiten Hohlzylinderendes (22) seitlich in den Einführschlitz (24) ragende und elastisch nachgiebige Klemmabschnitte (KA) aufweist, die zum elastischen Klemmen des Sensorelements (S) eingerichtet sind.

4. Haltevorrichtung (1) nach einem der vorhergehenden Ansprüche, wobei der Einführschlitz (24) im Bereich des ersten Hohlzylinderendes (21) und damit im Bereich des Befestigungsabschnitts (10) eine Verbreiterung (29) aufweist.

5. Haltevorrichtung (1) nach einem der vorhergehenden Ansprüche, wobei der Hohlzylinderabschnitt (20) und der Befestigungsabschnitt (10) einstückig miteinander verbunden sind.

6. Haltevorrichtung (1) nach Anspruch 5, wobei die Haltevorrichtung (1) einstückig aus Kunststoff (K) gefertigt ist.

7. Haltevorrichtung (1) nach Anspruch 5 oder 6, wobei der Hohlzylinderabschnitt (20) mittels einer Verrippung (30) an den Befestigungsabschnitt (10) angeschlossen ist.

8. Haltevorrichtung (1) nach Anspruch 7, wobei die Verrippung (30) mehrere Rippen (31, 32, 33) aufweist, die jeweils ausgehend von dem Befestigungsabschnitt (10) bis auf eine Außenfläche des Hohlzylinderabschnitts (20) axial längserstreckt sind.

9. Haltevorrichtung (1) nach Anspruch 7 oder 8, wobei die Verrippung (30) mehrere Rippen (31, 32, 33) aufweist, die in Umfangsrichtung des Hohlzylinderabschnitts (20) zueinander versetzt angeordnet sind.

10. Haltevorrichtung (1) nach einem der Ansprüche 5 bis 9, wobei der Hohlzylinderabschnitt (20) mittels einer Verrundung (40) an den Befestigungsabschnitt (10) angeschlossen ist.

11. Haltevorrichtung (1) nach Anspruch 10 in Kombination mit einem der Ansprüche 7 bis 9, wobei die Verrippung (30) auf der Verrundung (40) angeordnet ist.

12. Haltevorrichtung (1) nach einem der vorhergehenden Ansprüche, wobei der Befestigungsabschnitt (10) wenigstens einen Flanschabschnitt (11, 11') aufweist, der radial von dem ersten Hohlzylinderende (21) abragt und wenigstens eine Befestigungsbohrung (12, 12') zum Aufnehmen eines Befestigungsmittels aufweist.

13. Anordnung (A) mit einer Haltevorrichtung (1) nach einem der vorhergehenden Ansprüche und mit einem Sensorelement (S), das in der Haltebohrung (23) der Haltevorrichtung (1) gehalten ist.

14. Dialysegerät (D) mit einer Haltevorrichtung (1) nach einem der Ansprüche 1 bis 12 und/oder mit einer Anordnung (A) nach Anspruch 13.
